Europäisches Patentamt

European Patent Office

Office européen des brevets

(11) Publication number: **0 263 315**
**A2**

(12) **EUROPEAN PATENT APPLICATION**

(21) Application number: **87113208.0**

(51) Int. Cl.⁴: **A61F 5/44**

(22) Date of filing: **08.10.82**

(30) Priority: **08.10.81 JP 148806/81 U**
**19.12.81 JP 190176/81 U**
**27.05.82 JP 76981/82 U**

(43) Date of publication of application:
**13.04.88 Bulletin 88/15**

(60) Publication number of the earlier application in accordance with Art.76 EPC: **0 093 175**

(84) Designated Contracting States:
**DE FR GB**

(71) Applicant: **TERAUCHI, Ryugo**
**29-18, Minamiogikubo 4-chome**
**Suginami-ku Tokyo 167(JP)**

Applicant: **FUKUSHIMA, Kiyoshi**
**3-4, Kotesashi-cho Tokorozawa-shi**
**Saitama-ken 359(JP)**

(72) Inventor: **TERAUCHI, Ryugo**
**29-18, Minamiogikubo 4-chome**
**Suginami-ku Tokyo 167(JP)**
Inventor: **FUKUSHIMA, Kiyoshi**
**3-4, Kotesashi-cho Tokorozawa-shi**
**Saitama-ken 359(JP)**

(74) Representative: **Smith, Norman Ian et al**
**F.J. CLEVELAND & COMPANY 40-43**
**Chancery Lane**
**London WC2A 1JQ(GB)**

(54) **Urinary appliance.**

(57) This invention relates to a urinary appliance for use by incontinent patients. The urinary appliance of the present invention is equipped with a urine receiving bag 3 made of a soft and flexible fabric that is permeable to air, and water repellent. A urinal 8 or a urine storage bag 32 is connected to the urine receiving bag 3. A high water-absorbant resin 34 is placed inside the urine storage bag 32 so as to absorb and gel the urine.

Fig. 1

This is a division of the co-pending patent application Serial No. 82902988.3, filed on October 8, 1982.

This invention relates generally to a urinary appliance for use by the incontinent such as the aged, patients with spinal injuries and the like.

In general, urinary appliances have a urine receiving unit, which is applied to the patient's genitals, so as to receive the urine. A urinal or a urine receiving bag is connected to the urine receiving unit and stores the urine flowing from the urine receiving unit therein.

The conventional urine receiving unit is made of plastic or latex rubber and has the following problem. Since the inside of the urine receiving unit is cut off from the atmosphere and is kept sealed, moisture caused by the urine stays therein and the skin of the genitals can become irritated and sore because of this moisture. Moreover, the urine does not flow smoothly into the urinal or urine bag because the urine inside the urine receiving unit is not at atmospheric pressure. Furthermore, the plastic urine receiving unit is hard and inconvenient for the patient when walking.

It is an object of the present invention to provide a urinary appliance which alleviates some of the above problems.

According to one aspect of the present invention there is provided a urinary appliance comprising a urine receiving bag. Said urine receiving bag made of a soft and flexible fabric that is permeable to air, and water repellent, and having an opening to be fitted over the genitals of a female user of the appliance. The appliance is characterized by a urine outlet at a smaller end thereof. The urine receiving bag is treated with a water repellent agent. A ring is fitted around the periphery of said opening of said receiving unit bag. The ring is made of a soft and flexible material having shape retaining properties, and it is covered with a soft and flexible cloth fabric that is permeable to air and water repellent.

In an embodiment of a urinary appliance in accordance with the present invention, a urine storage bag incorporating therein a highly water-absorbent resin is connected to the urine receiving bag. The urine is absorbed by this water-absorbent resin, is changed into a gel and thus loses its fluidity. Hence, the patient or wearer of the bag can easily practice so-called "walking rehabilitation". The urine does not flow back from the urine storage bag to the urine receiving bag.

The invention will be described now by way of example only with particular reference to the accompanying drawings. In this drawings;

Figure 1 shows one embodiment of the present invention and is a perspective view illustrating the urinary appliance for a female incontinent patient when lying down;

Figure 2 is a front view of the device shown in Figure 1;

Figure 3 is a perspective view illustrating another urine receiving bag for a female incontinent patient in accordance with the present invention;

Figure 4 is a partly enlarged sectional view of the urine receiving bag shown in Figure 3; and

Figures 5 through 7 are partly cut-away front views, each illustrating another embodiment of the present invention, being designed so as to enable the patient to walk easily.

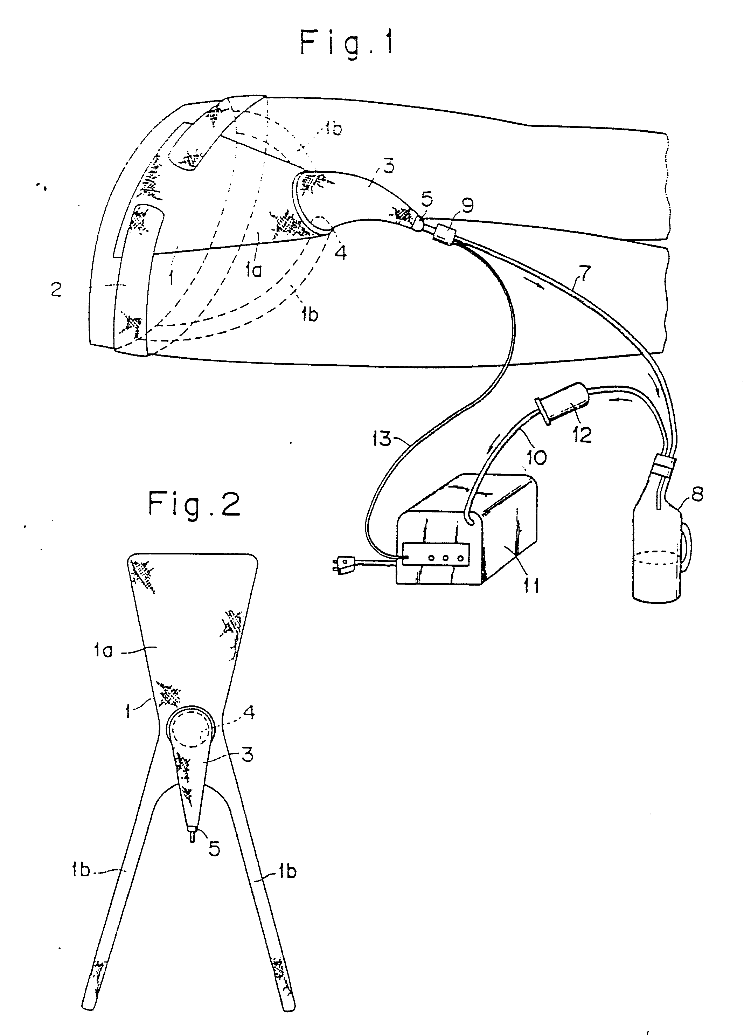

One embodiment of the percent invention will now be described with reference to Figures 1 and 2. The device 1 is shown fitted around the waist of a patient by a belt 2. The device 1 has a front section 1a which fits over the abdominal region of the patient, and two narrow elongated rear sections 1b, 1b which are positioned over the buttocks of the patient. These front and rear sections 1a, 1b, 1b are detachably fitted to the belt 2 by suitable means such as "magic tape" or Velcro. A urine receiving bag 3 is fitted to the front section 1a of the device 1. The urine receiving bag 3 has a shape suitable for a female. The urine receiving bag 3 has an opening 4 at one of its ends and a urine outlet 5 at the other. The periphery of this opening 4 is fitted to the device 1.

The urine receiving bag 3 is made of a soft and flexible fabric which is permeable to air, and water repellent. The fabric is a woven or non-woven fabric that has been treated with a water-repelling agent. If a woven fabric is used, it is possible to first treat the yarn prior to weaving with the water-repelling agent and then weave the yarn to obtain the woven fabric. The fabric forming the urine receiving bag may be made of a porous resin membrane having through pores. The device 1 may also be formed of the same fabric as that of the urine receiving bag 3.

A urinal 8 is connected to the urine outlet 5 of the urine receiving bag 3 via a tube 7. A sensor is fitted to the urine outlet 5, and a suction unit 11 is connected to the urine outlet 5 via another tube 10 with a deodorizing filter 12 interposed in the tube 10. The sensor 9 is electrically connected to the suction unit 11 via wires 13.

The operation of the urinary appliance having the construction described above will now be described. When the patient urinates when lying down, the urine is received by the urine receiving bag 3 and some of the urine reaches the sensor 9 through the urine outlet 5. The sensor 9 senses the urine and sends a detection signal to the suction unit 11. The suction unit 11 is actuated by this signal so that the air inside the urinal 8 is exhausted through an exhaust port (not shown) formed in the suction unit 11 through the tube 10. Since the inside of the urinal 8 is kept at a negative pressure in this case, the urine inside the urine receiving bag 3 is forced to flow into the urinal 8.

Since the urine receiving bag 3 is permeable to air, its interior is always at atmospheric pressure so that the urine inside the urine receiving bag 3 flows smoothly into the urinal 8 when the suction unit 11 is actuated as described above. Also, because the urine receiving bag 3 is permeable to air, the moisture generated by the urine does not stay inside the urine receiving bag 3 but evaporates outside through the surface of urine receiving bag 3. Thus, the skin of the patient can be prevented from becoming sore.

Since the urine receiving bag 3 is also water repellent, the urine neither leaks nor soaks in. Since the urine receiving bag is soft and flexible, it does not feel offensive to the patient.

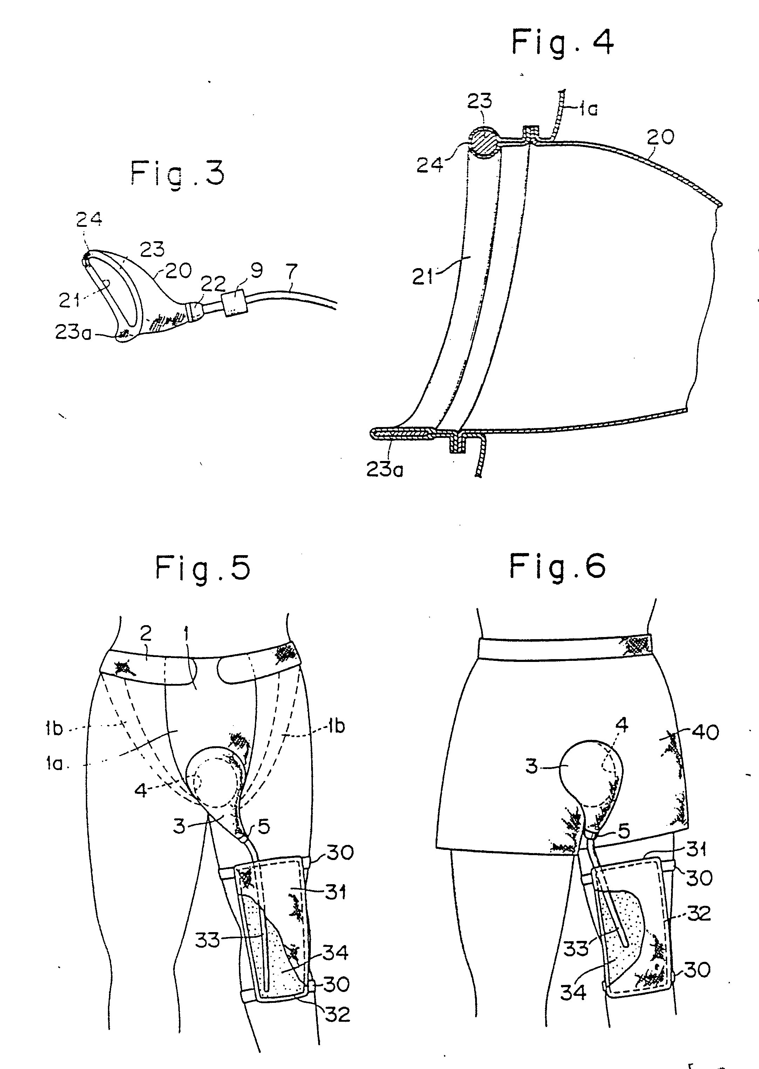

Figures 3 and 4 illustrate another form of a urine receiving bag 20 for a female incontinent patient. In the same way as the urine receiving bag 3 as shown in Figure 1, this urine receiving bag 20 is made of a soft and flexible fabric that is permeable to air, and water repellent, and has an opening 21 and a urine outlet 22. The opening 21 has a relatively large area and a ring 23 is fitted around the opening. The ring 23 is made of a soft and flexible material having good shape retaining properties such as foam rubber, urethane foam, or the like. It is shaped in such a manner that it comes into contact with the periphery of the female genitals. The ring 23 has at one part of a flat tongue 23a and the rest of the ring is shaped to be round in cross-section. The tongue 23a is fitted to the permineum of the female genitals. The ring 23 is covered with a soft and flexible fabric 24 which is permeable to air and water repellent.

The urine receiving unit 3 as well as the urine receiving unit 20 may be detachably fitted to the device 1. The urine receiving unit 3 has a generally funnel shape whereas the unit 20 has a shape which has more of a cup shape.

In the following description of Figures 5 through 7 we refer to bag 3. It is understood this is also intended to include bag 20.

Figures 5 through 7 each show further embodiments of the present invention. These embodiments are specifically designed so that the incontinent patient can walk more easily.

In Figure 5, an external bag 31 is shown fitted to one of the legs of the patient by a band 30. This external bag 31 is made of cloth, plastic or the like. A cloth bag is preferred in this embodiment because the bag comes into direct contact with the skin. The external bag 31 is open at its upper part so that it receives a urine storage bag 32. The urine storage bag 32 is made of rubber or plastic. A tube 33 connected to the urine outlet 5 of the urine receiving bag 3 is inserted into the urine storage bag 32 and is sealed in place. A highly water absorbent resin 34 is placed inside the urine storage bag 32. The following can be listed as examples of the highly water absorbent resin;

(1) starch-acrylonitrile grafted polymer types of highly water-absorbent resins;

(2) cellulose-acrylonitrile grafted polymer types of highly water-absorbent resins;

(3) carboxymethylated polysaccharide-acrylonitrile grafted polymer types of highly water-absorbent resins;

(4) polyacrylic acid types of highly water-absorbent resins;

(5) polyacrylonitrile of highly water-absorbent resins;

(6) non-ionic polymer types of highly water-absorbent resins.

The highly water-absorbent resins may assume various forms such as fibers, cloth, cotton, powder, and so on. The water-absorbent resins 34 may be wrapped in craft paper or the like and is then placed in the urine storage bag 32.

The operation of the urinary appliance shown in Figure 5 will now be described. When the patient urinates when standing, the urine is received by the urine receiving bag 3 and flows into the urine storage bag 32 via the tube 33 due to gravitational force. Since the urine receiving bag 3 is permeable to air, the urine inside the urine receiving bag 3 is at atmospheric pressure and flows smoothly into the urine storage bag 32. The urine is then absorbed by the highly water absorbent resin 34 inside the urine storage bag 32, is changed into a gel and thus loses its fluidity. The patient can walk easily because the urine receiving bag 3 is soft and flexible and because the urine is the form of a gel inside the urine storage bag 32 and no longer flows.

Even if there are small holes in the urine storage bag 32, the gelled urine will neither leak nor flow back to the urine receiving bag 31. The highly water-absorbent resin can also absorb ammonia released by the decomposition of urine and can thus prevent the occurrence of offensive odors.

The gelled urine is removed from the urine storage 32 and is then discarded. The urine storage bag 32 itself may be disposed.

In Figure 6, the urine receiving bag 3 is shown fitted to underpants 40 similar to underpants which are adapted to accept the receiving bag. This type of underpants is not normal for females and in this case females should use special underpants or underpants which are normally used by males.

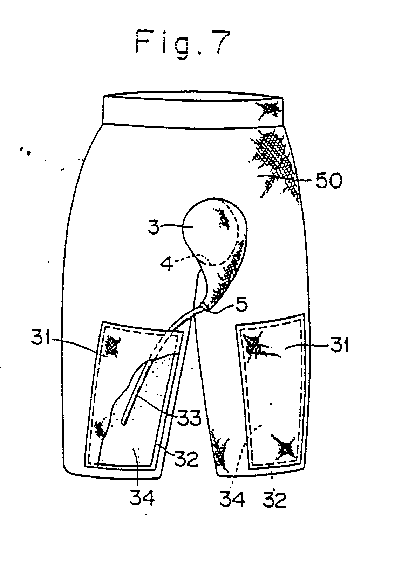

In Figure 7, the urine receiving bag 3 is shown fitted to elongated underpants 50 and two external bags 31, 31 are also fitted to the underpants 50. Each external bag 31 has placed therein a urine storage bag 32 which in turn has placed therein the highly water-absorbent resin 34. One of the urine storage bags 32 is a spare.

## Claims

1. A urinary appliance comprising a urine receiving bag (3, 20), said urine receiving bag (3, 20) is made of a soft and flexible fabric that is permeable to air, and water repellent, and has an opening (4, 21) to be fitted over the genitals of a female user of the appliance, characterized by a urine outlet (5, 22) at a smaller end thereof; said urine receiving bag (3, 20) is treated with a water repellent agent; a ring (23) is fitted around the periphery of said opening (4, 21) of said receiving unit bag (3), and said ring (23) is made of a soft and flexible material having shape retaining properties, and is covered with a soft and flexible fabric (24) that is permeable to air and water repellent.

2. A urinary appliance according to claim 2 characterized in that said ring (23) has a tongue (23a) at one part and said tongue (23a) is placed against the perineum of the female genitals.

3. A urinary appliance according to claim 1 or 2 characterized by further including a body engageable portion (1).

4. A urinary appliance according to claim 1, 2 or 3 characterized in that said body engageable portion (1) includes a belt (2).

5. A urinary appliance according to claim 4, characterized in that said body engaging portion (1) includes a front section (1a) which fits over the abdominal region of the female patient.

6. A urinary appliance according to any one of claims 1 through 5, characterized in that said flexible material of said urine receiving bag (20) is made of woven fabric material.

7. A urinary appliance according to any one of claims 1 through 5, characterized in that said flexible material of said urine receiving bag (20) is made of unwoven porous membrane.

8. A urinary appliance according to any one of claims 1 through 7, further characterized in that said urine outlet (22) is connected to a sensor (9).

9. A urinary appliance according to any one of claims 1 through 8, characterized in that said sensor (9) is connected to a tube (7).

10. A urinary appliance according to any one of claims 1 through 9, characterized in that said urine outlet (22) is connected to a tube (7).

11. A urinary appliance according to any one of claims 1 through 10, characterized in that said ring (23) is deformed arcuately to engage the periphery of the female genitals.

12. A urinary appliance according to any one of claims 1 through 11, characterized in that a urine storage bag (32) is connected to said urine outlet (22) of said urine receiving bag (20).

13. A urinary appliance according to claim 12 characterized in that a highly water absorbent resin (54) which absorbs and gels the urine is placed inside said urine storage bag (32).

14. A urinary appliance according to claim 1 characterized in that a urinal (8) is connected to said urine outlet (22) of said urine storage bag (20) via a tube (7), a suction unit (11) is connected to said urinal (8) via another tube (10), and a sensor (9) is further fitted to said urine outlet (22) so that when said sensor (9) senses the urine, it actuates said suction unit (11) to extract the air inside said urinal (8) and thereby forces the urine inside said urine receiving bag (3), said urine receiving bag (20) to flow into said urinal (8).

15. A urinary appliance according to claim 1 characterized in that the urine receiving bag (30) is adapted to be removably attached to conventional cloth undergarments (40).

16. A urinary appliance according to claim 15 characterized in that at least one urine storage bag (31) is connected to said urine outlet (22) of said urine receiving bag (20).

17. A urinary appliance according to claim 1, characterized in that the urine storage bag (3) is substantially funnel shaped.

18. A urinary appliance according to claim 1, characterized in that the urine receiving bag (20) is substantially cup-shaped.

# Fig.1

# Fig.2

Fig. 4

Fig. 3

Fig. 5

Fig. 6

# Fig.7